# DEMANDE DE BREVET EUROPEEN

(11) **EP 4 614 128 A1**
(43) Date de publication de la demande: **10.09.2025**
(21) Numéro de dépôt: 25158217.7
(22) Date de dépôt: 17.02.2025
(51) Int. Cl.: G01L 19/12

(54) **BOITIER DE SURVEILLANCE D'UN RÉSEAU HOSPITALIER DE FLUIDES AVEC BOUTONS À FONCTIONS MULTIPLES**

(30) Priorité: 07.03.2024 FR 2402294
(71) Demandeur: Air Liquide Medical Systems, 92182 Antony Cedex (FR)
(72) Inventeur: BUSSON, Thibaut, 92182 Antony (FR)
(74) Mandataire: Air Liquide

(57) **Abrégé**

L'invention concerne une installation (1) d'acheminement de fluide, tel des gaz ou du vide, dans un établissement hospitalier, comprenant des lignes d'acheminement de fluide (14-1, 14-2, 14-3) avec des capteurs de pression (5), et un boitier de surveillance de fluide (3) et des moyens d'alarme (35). La partie de boitier avant (11) comprend des moyens de sélection de mode (18) agencés dans le compartiment interne (32) du boitier (3) et actionnables par l'utilisateur pour activer un mode dit « de fonctionnement normal » ou un mode dit « de maintenance ». Une ou des touches de contrôle (21, 22) permettent, selon le mode sélectionné, de stopper une alarme sonore ayant été déclenchée par les moyens d'alarme (35) ou de tester un bon fonctionnement des moyens d'alarme (35), et de sélectionner ou fixer au moins un paramètre de configuration.

## Description

L'invention concerne une installation d'acheminement de fluide dans un établissement hospitalier équipée d'un boitier ou appareil de surveillance de fluides médicaux, tels les gaz ou le vide médical (i.e. dépression).

Les boîtiers de surveillance des fluides médicaux sont des éléments de sécurité obligatoires qui permettent de surveiller, i.e. monitorer, les réseaux de distribution des fluides médicaux, typiquement des lignes ou canalisations d'acheminement des gaz médicaux, tel de l'air, de l'oxygène ou du protoxyde d'azote (N₂O), voire du dioxyde de carbone (CO₂), ou du vide (i.e. dépression), agencés dans les établissements hospitaliers, tels les hôpitaux, cliniques ou analogues.

Un boîtier de surveillance est habituellement installé sur un mur, une paroi ou analogue, de l'établissement hospitalier considéré, par exemple dans la ou les zones de production des fluides médicaux et/ou à l'entrée d'un ou plusieurs services hospitaliers. Des capteurs de pression reliés aux différentes lignes du réseau de fluides médicaux à surveiller sont connectés électriquement au boîtier de surveillance, en particulier aux moyens de pilotage du boîtier auxquels les capteurs fournissent les mesures de pression opérées afin qu'elles y soient traitées informatiquement.

Chaque capteur de pression est relié à une voie de surveillance du boîtier de surveillance qui est paramétrée de manière spécifique. Plus précisément, afin d'assurer une surveillance correcte du réseau de fluides, il est indispensable de définir les paramètres caractérisant chaque voie de surveillance du boitier afin de pouvoir ensuite déclencher une alarme lorsqu'une anomalie est détectée, typiquement un franchissement de valeur-seuil de pression d'alerte par exemple.

Or, la programmation du boîtier varie en fonction des différents paramètres à surveiller, notamment en fonction du type de fluide surveillé (i.e. oxygène, air, protoxyde d'azote ou vide, i.e. dépression), du type de capteur (i.e. ses caractéristiques intrinsèques), des niveaux de pression à surveiller.... mais aussi en fonction de l'utilisation du boîtier, par exemple pour surveiller une production de fluides ou une alimentation en fluides. La programmation ou paramétrage du boîtier se fait habituellement au moment de la mise en service du boîtier de surveillance, c'est-à-dire lors de son installation dans l'hôpital ou analogue. Parfois, une (re)programmation peut aussi être nécessaire après mise en service, par exemple après quelques mois, typiquement en cas de modifications faisant suite à une opération de maintenance ou analogue.

Pour des raisons évidentes, il est souhaitable que le paramétrage puisse se faire de manière aussi simple possible mais qu'après configuration du boitier, il ne soit pas possible, pour des personnes non-habilitées, tel le personnel hospitalier, de modifier le paramétrage du boitier. Le paramétrage doit aussi être possible sans engendrer de déclenchement d'alarme intempestif.

Par ailleurs, il doit être possible de vérifier facilement le bon fonctionnement du boitier, en particulier d'une alarme sonore, mais sans donner accès aux éléments internes du boitier afin d'éviter de mauvaises manipulations pouvant conduire à un déréglage du paramétrage.

US2006/290525 enseigne une installation d'acheminement de fluides dans un établissement hospitalier comprenant un boitier équipé de touches de sélection externes permettant d'effectuer des choix ou sélection, ou de lancer des tests de fonctionnement, mais rien de plus, en particulier il n'offre pas une ergonomie très adaptée à la maintenance, à l'installation et à la programmation.

Un problème est dès lors de proposer une installation d'acheminement de fluides dans un établissement hospitalier améliorée, en particulier de permettre et/ou faciliter le paramétrage d'un boitier de surveillance de fluide utilisé dans l'installation, notamment lors de la mise en route du boitier, typiquement à la fin d'un chantier d'installation d'un réseau de fluides, i.e. gaz médicaux et/ou vide médical, tout en évitant tout ou partie des problèmes susmentionnés.

Une solution concerne alors une installation d'acheminement de fluide (i.e. un ou plusieurs fluides) dans un établissement hospitalier, comprenant :
- des lignes d'acheminement de fluide comprenant chacune un capteur de pression, et
- un boitier de surveillance de fluide comprenant une partie de boitier arrière et une partie de boitier avant définissant entre elles un compartiment interne du boitier, et des moyens d'alarme, dans lequel :
   ∘ **ladite partie** de boitier avant est montée pivotante (i.e. articulée) sur la partie de boitier arrière entre au moins une position d'ouverture dans laquelle un utilisateur a accès au compartiment interne et une position de fermeture dans laquelle l'utilisateur n'a pas accès au compartiment interne,
   ∘ et la partie de boitier avant comprend :
      ▪ du côté interne, c'est-à-dire du côté du compartiment interne, des moyens de pilotage à processeur configurés pour opérer une surveillance de la pression dans les lignes d'acheminement de fluide en traitant les mesures de pression provenant des capteurs de pression et
      ▪ en façade, c'est-à-dire du côté extérieur, au moins une ou touche de contrôle à actionnement par pression digitale de l'utilisateur, par exemple un appui de l'index.

De plus, dans l'installation de l'invention, la partie de boitier avant comprend en outre des moyens de sélection de mode agencés dans le compartiment interne du boitier et actionnables par l'utilisateur pour activer au moins deux modes de fonctionement, comprenant :
▪ un mode dit « de fonctionnement normal », dans lequel les moyens de pilotage sont configurés pour activer les moyens d'alarme et déclencher au moins une alarme sonore en cas de détection d'une pression inadéquate dans l'une ou plusieurs des lignes d'acheminement de fluide, ou
▪ un mode dit « de maintenance », dans lequel les moyens de pilotage sont configurés pour contrôler les moyens d'alarme pour empêcher tout déclenchement d'alarme sonore tant que le mode dit « de maintenance » est activé.

Par ailleurs, ladite au moins une touche de contrôle est configurée pour :
▪ lorsque le mode « de fonctionnement normal » a été activé, permettre à l'utilisateur de stopper une alarme sonore ayant été déclenchée par les moyens d'alarme ou de tester un bon fonctionnement des moyens d'alarme sonore, et
▪ **lorsque** le mode « de maintenance » a été activé, permettre à l'utilisateur de sélectionner ou fixer au moins un paramètre de configuration.

Selon le mode de réalisation considéré, le boîtier et/ou l'installation de l'invention peut/peuvent comprendre l'une ou plusieurs des caractéristiques suivantes:
- ladite au moins une touche de contrôle est en outre configurée permettre à l'utilisateur de choisir une langue d'affichage lorsque le mode « maintenance » est activé, par exemple une langue choisie parmi le français, l'anglais, l'allemand, l'espagnol, l'italien, le portugais, l'arabe ou une autre langue adaptée.
- les moyens d'alarme sont configurés pour déclencher une alarme sonore et une alarme visuelle.
- la partie de boitier arrière est fixée à une paroi de l'établissement hospitalier, par exemple un mur, une cloison ou analogue.
- les moyens de sélection de mode du boitier comprennent un bouton, tel un bouton-poussoir, un contacteur basculant (i.e. switch) ou analogue.
- les moyens de sélection de mode sont agencés sur une carte électronique faisant partie des moyens de pilotage
- la carte électronique du boitier comprend le (micro)processeur.
- au moins deux touches de contrôle à actionnement par pression digitale de l'utilisateur sont agencées sur la façade du boitier.
- les deux touches de contrôle comprennent une première touche de contrôle et une seconde touche de contrôle.
- la première touche de contrôle est configurée pour permettre à l'utilisateur de stopper une alarme sonore ayant été déclenchée par les moyens d'alarme lorsque le mode « de fonctionnement normal » est activé.
- la première touche de contrôle est configurée pour permettre à l'utilisateur de sélectionner ou fixer au moins un paramètre de configuration du boitier lorsque le mode « maintenance » est activé.
- la seconde touche de contrôle est configurée pour permettre à l'utilisateur de tester un bon fonctionnement des moyens d'alarme, en particulier l'alarme sonore, lorsque le mode « de fonctionnement normal » est activé.
- **la seconde** touche de contrôle est configurée pour permettre à l'utilisateur de choisir une langue d'affichage lorsque le mode « maintenance » est activé.
- ledit au moins un paramètre de configuration du boitier est choisi parmi un type de fluide, un type de capteur de pression, un seuil de pression basse et un seuil de pression haute.
- la première touche de contrôle est configurée pour permettre à l'utilisateur de sélectionner ou fixer plusieurs paramètres de configuration, en particulier un ensemble de paramètres de configuration, lorsque le mode « maintenance » est activé.
- lorsque le mode « maintenance » est activé, les moyens de pilotage sont configurés pour commander les moyens d'alarme pour délivrer un signal sonore à une fréquence donnée, typiquement toutes les 1 à 10 minutes, typiquement toutes les 3 à 7 minutes, en particulier un « bip » sonore permettant de signaler que le boitier est en mode « maintenance ».
- les moyens d'alarme sonore comprennent un buzzer.
- les moyens de pilotage sont configurés pour continuer à opérer une surveillance de la pression dans les lignes d'acheminement de fluide, y compris lorsque le mode « maintenance » est activé.
- lorsque le mode « maintenance » est activé, les moyens d'alarme sont configurés pour déclencher ou délivrer une alarme visuelle de manière à signaler à l'utilisateur que le boitier est en mode « maintenance » ou qu'une pression inadéquate a été détectée par les moyens de pilotage.
- les lignes d'acheminement de fluide sont choisies parmi au moins une ligne d'oxygène, une ligne d'air et une ligne de vide, voire une ligne de protoxyde d'azote et/ou une ligne de CO₂.
- le boitier de surveillance de fluide et les lignes d'acheminement de fluide sont agencés sur une ou des parois de l'établissement hospitalier.
- la partie de boitier avant comprend des moyens d'affichage agencées en façade, c'est-à-dire du côté extérieur, par exemple un écran d'affichage, de préférence en couleurs.
- les moyens d'alarme coopérant avec les moyens d'affichage pour afficher ou déclencher une alarme visuelle, simultanément au déclenchement d'une alarme sonore.
- les moyens d'alarme sont contrôlées par les moyens de pilotage.

Selon le mode de réalisation considéré, le boîtier et/ou l'installation selon l'invention peut comprendre l'une ou plusieurs des caractéristiques suivantes :
- le boitier comprend plusieurs voies de surveillance comprenant chacune des moyens de raccordement électrique à un capteur de pression, les voies de surveillance étant reliées électriquement aux capteurs de pression, via des moyens de raccordement électrique et des liaisons électriques.
- des moyens de pilotage à processeur coopérant avec les voies de surveillance pour traiter des mesures de pression provenant des capteurs de pression.
- les moyens de pilotage à processeur comprennent un ou plusieurs (micro)processeur(s).
- le ou les (micro)processeur(s) sont agencés sur (au moins) une carte électronique.
- les moyens de pilotage à processeur comprennent un microcontrôleur.
- la (les) carte(s) électronique(s) est (sont) agencée(s) dans la partie avant de boitier, i.e. du côté intérieur.
- les moyens d'alarme sonore sont commandés par les moyens de pilotage.
- l'alarme visuelle comprend un signal lumineux, par exemple une lumière clignotante ou fixe, et/ou un affichage sur les moyens d'affichage d'un message d'alarme ou d'un pictogramme d'alarme, ou toute autre information.
- les moyens d'affichage sont configurés pour afficher toute information dans la langue sélectionnée.
- le boitier comprend des moyens de mémorisation configurés pour mémoriser plusieurs ensembles (Ei) de paramètres de configuration, où chaque ensemble (Ei) de paramètres de configuration comprend plusieurs paramètres de configuration choisis parmi un type de fluide, un type de capteur de pression, un seuil de pression basse et un seuil de pression haute.
- des moyens de sélection configurés pour permettre à un opérateur de choisir au moins l'un desdits ensembles (Ei) de paramètres de configuration mémorisés et de l'associer spécifiquement à l'une des voies de surveillance.
- la partie de boitier avant est montée pivotante (i.e. articulée) sur la partie de boitier arrière autour d'un axe de pivotement horizontal, typiquement une charnière ou analogue.
- la partie de boitier avant bascule autour de l'axe de pivotement horizontal du boitier jusqu'à atteindre la position d'ouverture.
- la partie de boitier avant bascule autour de l'axe de pivotement horizontal du boitier d'un angle compris entre 120 et 180° environ.
- l'axe de pivotement horizontal du boitier est agencé en bas de boitier, typiquement au niveau du rebord inférieur de la partie arrière de boitier, lorsque la partie arrière de boitier est considérée agencée sur une paroi, c'est-à-dire au niveau du rebord de la partie arrière de boitier situé le plus proche du sol.
- en position de fermeture, la partie de boitier avant recouvre et ferme la partie de boitier arrière, c'est-à-dire qu'un utilisateur n'a pas accès au compartiment interne du boitier.
- le boitier comprend des moyens de verrouillage configurés pour permettre de verrouiller la partie de boitier avant sur la partie de boitier arrière pour empêcher une personne non-habilitée de la mettre dans la position d'ouverture, c'est-à-dire d'empêcher tout accès au compartiment interne.
- les moyens de verrouillage peuvent être déverrouillés pour libérer la partie avant de boitier et autoriser le passage en position d'ouverture grâce à un moyen de déverrouillage dédié, tel un outil, une clé, un code ou analogue.
- les moyens de verrouillage comprennent un mécanisme d'ouverture et/ou de fermeture.
- le mécanisme d'ouverture et/ou de fermeture est configuré pour être actionnable au moyen d'un outil dédié ou d'une clé afin d'opérer un verrouillage ou un déverrouillage.
- alternativement, le mécanisme d'ouverture et/ou de fermeture comprend un système de verrouillage/déverrouillage par code confidentiel ou biométrique.
- les voies de surveillance comprennent des liaisons électriques reliant les moyens de pilotage aux moyens de raccordement électrique, par exemple des liaisons filaires, de type circuit imprimé ou autres.
- la partie arrière du boitier comprend un ou plusieurs entrées ou ouvertures dimensionnées pour permettre le passage des liaisons électriques, tels les câbles ou autres.
- les parties avant et arrière du boitier forment un coffret rigide, en particulier en position de fermeture.
- les parties avant et arrière du boitier sont en polymère ou un autre matériau adapté.
- les moyens de pilotage à processeur comprennent un ou plusieurs (micro)processeur(s).
- les moyens de mémorisation comprennent une mémoire informatique, telle une mémoire flash, RAM ou autre.
- les moyens de mémorisation sont agencés sur la (les) carte électronique.
- selon un mode de réalisation, la ou une carte électronique comprend au moins une prise de raccordement de type USB permettant d'y raccorder un appareil externe, via un câble USB complémentaire ou analogue, par exemple un appareil externe permettant d'opérer un paramétrage avancé ou autre.
- les moyens de pilotage sont configurés pour comparer les mesures de pression provenant des différentes voies de surveillance avec les seuils de pression basse et/ou de pression haute des paramètres de configuration associés aux différentes voies de surveillance et mémorisés par les moyens de mémorisation de données afin de déclencher une alarme sonore et/ou une alarme visuelle lorsqu'une pression inadéquate ou non-conforme est détectée, c'est-à-dire typiquement une pression excessive ou insuffisante par rapport aux seuils de pression basse et/ou de pression haute.
- les moyens de pilotage sont configurés pour déterminer qu'une pression mesurée est inadéquate et/ou non-conforme et correspond à une pression excessive lorsque ladite pression mesurée est supérieure à un seuil de pression haute donné, i.e. mémorisé.
- les moyens de pilotage sont configurés pour déterminer qu'une pression mesurée inadéquate et/ou non-conforme et correspond à une pression insuffisante lorsque ladite pression mesurée est inférieure à un seuil de pression basse donné, i.e. mémorisé.
- les moyens de raccordement électrique du boitier comprennent des connecteurs électriques ou analogues, tel qu'un bornier électrique ou analogue, c'est-à-dire plusieurs emplacements de raccordement électrique.
- chaque emplacement de raccordement électrique du boitier est configuré pour y raccorder un capteur de pression.
- le boitier comprend de 2 à 10 voies de surveillance, de préférence de 3 à 8 voies de surveillance, par exemple 4 voies.
- les voies de surveillance sont agencées en parallèle les unes des autres.
- chaque ou les ensembles (Ei) de paramètres de configuration comprennent en tant que paramètre de configuration un type de fluide choisi parmi au moins l'oxygène, l'air et/ou le vide (i.e. dépression), voire du protoxyde d'azote et/ou dioxyde de carbone (CO₂) et/ou un autre gaz médical, typiquement de l'oxygène ou de l'air de qualité médicale.
- les lignes d'acheminement de fluide sont choisies parmi au moins une ligne d'oxygène, une ligne d'air et une ligne de vide, et éventuellement une ligne protoxyde d'azote et/ou une ligne de dioxyde de carbone (CO₂) et/ou d'un autre gaz médical.
- le boitier de surveillance de fluide et les lignes d'acheminement de fluide sont agencées au sein de l'établissement hospitalier, en particulier montés sur une ou des parois dudit établissement hospitalier, c'est-à-dire un mur, une cloison, un plafond ou autre.
- les ensembles (Ei) de paramètres de configuration comprennent en tant que paramètre de configuration, pour un gaz sous pression, un seuil de pression basse compris entre 3 et 7 bar abs et un seuil de pression haute compris entre 4.5 et 10 bar abs.
- les ensembles (Ei) de paramètres de configuration comprennent en tant que paramètre de configuration, pour du vide (dépression), un seuil de pression basse compris entre -0.8 et -0.3 bar abs et un seuil de pression haute compris entre -0.1 et 0.5 bar abs.
- les ensemble (Ei) de paramètres de configuration comprennent en tant que paramètre de configuration, un type de capteur choisi parmi un capteur 0 à 16 bar, un capteur 0 à 250 bars et un capteur 0 à -0,90 bar.
- alternativement, les ensemble (Ei) de paramètres de configuration comprennent en tant que paramètre de configuration, un type de capteur choisi parmi les capteurs tout ou rien.
- alternativement, les ensembles (Ei) de paramètres de configuration comprennent en tant que paramètre d'une source de production de gaz, un seuil de pression basse compris entre 15 et 25 bar abs, par exemple de l'ordre de 17 bar abs, ou un seuil de pression haute compris entre 110 et 150 bar abs, par exemple de l'ordre de 130 bar abs.
- les moyens de sélection sont configurés pour permettre à l'opérateur de choisir plusieurs ensembles (Ei) de paramètres de configuration mémorisés et d'associer lesdits ensembles (Ei) de paramètres de configuration spécifiquement à des voies de surveillance dédiées.
- les moyens d'affichage sont configurés pour afficher des données ou informations utiles à l'opérateur, en particulier les paramètres de configuration, les ensemble (Ei) de paramètres de configuration, les différentes voies, une ou des (seuils) alarmes ou autres.
- les moyens d'affichage comprennent un écran d'affichage.
- l'écran d'affichage est en couleurs ou en noir et blanc.
- selon un mode de réalisation particulier, l'écran d'affichage peut être à dalle tactile.
- les moyens de pilotage sont configurés pour commander ou contrôler le ou les affichages sur les moyens d'affichage.
- les moyens de pilotage sont reliés électriquement aux moyens d'affichage, par exemple via une nappe électrique ou autre.
- les moyens d'alarme sonore sont configurés pour émettre un (des) signal sonore audible par l'oreille humaine lorsqu'ils sont déclenchés.
- les moyens d'alarme sonore comprennent un dispositif à haut-parleur, typiquement de type buzzer, intégré au boîtier.
- les moyens d'alarme visuelle sont configurés pour émettre un (des) signal visuel, par exemple une alerte visuelle affichée sur l'écran d'affichage et/ou un signal lumineux émis par une (ou des) diode (LED) du boîtier.
- il comprend des moyens d'alimentation électrique alimentant les éléments nécessitant du courant électrique pour fonctionner, notamment les moyens de pilotage et/ou les moyens d'affichage et/ou les moyens d'alarme.
- les moyens d'alimentation électrique comprennent des moyens de raccordement au secteur (110/220V), en particulier un ou des câbles d'alimentation électrique.
- les capteurs de pression fournissent des mesures de la pression ou dépression (i.e. vide) mesurées dans les lignes d'acheminement de fluide, aux moyens de pilotage qui traitent ces mesures afin de déclencher une alarme en cas de détection d'une pression inadéquate, voire d'un autre problème, en particulier une pression excessive ou insuffisante.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation d'acheminement de fluide comprenant un boîtier de surveillance selon l'invention.
Fig. 2 schématise un mode de réalisation de la façade avant d'un boîtier selon l'invention.
Fig. 3 schématise un boîtier selon l'invention avec partie de boitier avant en position d'ouverture.

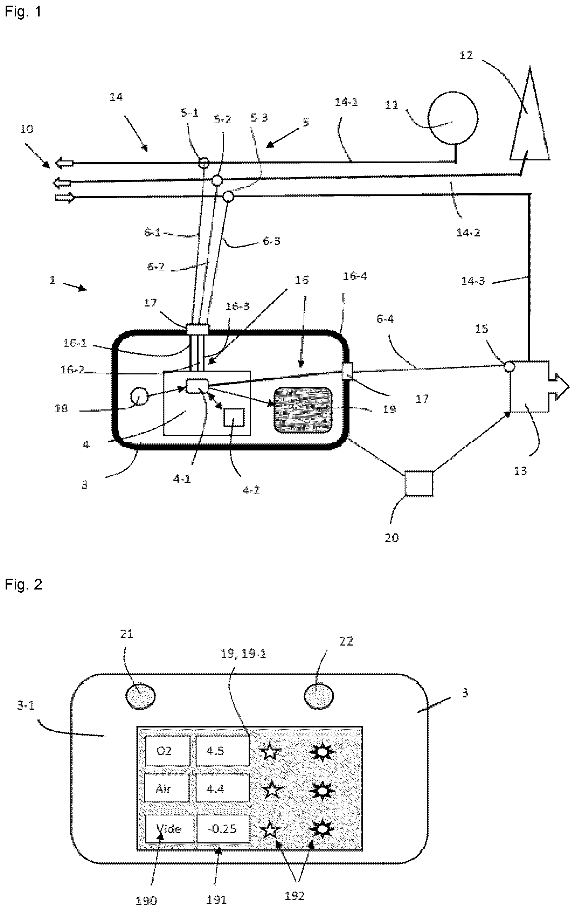

Fig. 1 schématise un mode de réalisation d'une installation 1 d'acheminement de fluide selon l'invention comprenant plusieurs lignes d'acheminement de fluide 14-1, 14-2, 14-3 faisant partie d'un réseau de canalisations 14 de fluides agencé au sein d'un établissement hospitalier, tel un hôpital, une clinique ou analogue. Par exemple, une ligne d'acheminement d'oxygène 14-1, une ligne d'acheminement d'air médical 14-2 et une ligne d'acheminement de vide 14-3, c'est-à-dire de dépression (i.e. < 1 atm).

Le réseau 14 de canalisations permet d'acheminer les fluides médicaux, i.e. ici des gaz et du vide (dépression), depuis leur site de production ou de stockage sur le site de l'établissement hospitalier, jusqu'à des prises de distribution murales 10 agencées dans leurs lieux d'utilisation au sein de l'établissement hospitalier, telles les salles de soins, d'opération ou de réveil, les urgences, les chambres ou autres.

Ainsi, l'air de qualité médicale peut être produit directement sur site au moyen d'une unité de type PSA air 12, comme décrit par EP-A-864818, et il en va de même du vide qui peut aussi être produit sur site au moyen d'une (ou plusieurs) pompe à vide 13, comme décrit par EP-A-1026567.

Par ailleurs, l'oxygène ou d'autres gaz, comme le N₂O ou le CO₂, peuvent être produits hors site, avant d'y être amenés par camion-citerne ou analogue, où ils sont stockés dans des réservoirs de stockage 11, ou des bouteilles de gaz qui peuvent être agencées en cadre réunissant plusieurs bouteilles reliées entre elles.

Alternativement, l'oxygène peut aussi être produit sur site par unité de type VSA O₂. De façon connue, une unité PSA (pressure swing adsorption) permet de produire du gaz par modulation de pression, alors qu'une unité VSA (vacuum swing adsorption) permet de produire du gaz par modulation de vide.

Les lignes d'acheminement de fluide 14-1, 14-2, 14-3 du réseau de canalisations 14 sont habituellement fixées sur les murs ou parois de l'établissement hospitalier. Il en va de même des prises murales 10 situées aux extrémités libres de ces lignes d'acheminement de fluide, comme décrit par EP-A-3719377.

L'installation 1 comprend par ailleurs un boitier de surveillance 3 du réseau 14 formé de deux demi-boitiers 30, 31, à savoir d'une partie de boitier arrière 30 et une partie de boitier avant 31 définissant entre elles un compartiment interne 32 du boitier 3.

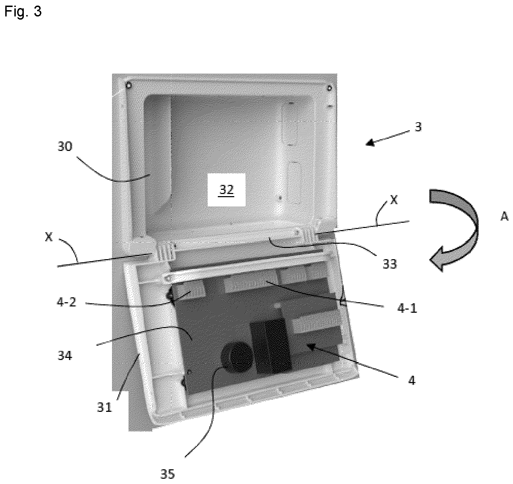

La partie de boitier avant 31 est montée pivotante, i.e. articulée, sur la partie de boitier arrière 10, typiquement autour d'un axe XX horizontal porté par la bordure inférieure 33 du boitier 3, entre au moins une position d'ouverture dans laquelle un utilisateur a accès au compartiment interne 32, comme illustré en Fig. 3, et une position de fermeture dans laquelle l'utilisateur n'a pas accès au compartiment interne 32, avec la partie de boitier avant 31 recouvrant la partie de boitier arrière 30 à la manière d'un couvercle.

Comme illustré en Fig. 3, le basculement de la partie de boitier avant 31 pour passer de la position de fermeture à la position d'ouverture (cf. Fig. 3), peut se faire selon un angle A compris entre environ 120° et 180°, de préférence entre 140° et 180° environ, ce qui donne un accès aisé aux éléments présents du côté interne 3-2 de la partie avant 31 du boitier 3, typiquement aux moyens de pilotages 4 (i.e. carte électronique 34, microprocesseur 4-1, mémoire 4-2...), aux moyens d'alarme sonore 35, aux voies 16 ; 16-1...16-4, c'est-à-dire les liaisons électriques servant au raccordement de moyens de mesure 5....

La partie de boitier avant 31 comprend, de son côté interne 31-1, des moyens de pilotage 4 comprenant un (ou plusieurs) (micro) processeur(s) 4-1 mettant en œuvre un ou des algorithmes pour traiter des mesures, informations, données, ou autres, et/ou déclencher une ou des alarmes comme expliqué ci-après. Le boîtier surveillance 3 forme un coffret rigide, par exemple en polymère, qui est fixé à une paroi de l'établissement hospitalier.

Les moyens de pilotage 4 peuvent aussi être appelés unité de pilotage, unité ou moyens de traitement d'information, électronique embarquée, contrôleur ou analogue. Ils comprennent typiquement une carte électronique 34 portant le (ou les) (micro) processeur(s) 4-1 et avantageusement des moyens de mémorisation 4-2, telle une mémoire flash ou analogue, servant à mémoriser ou stocker des paramètres de configuration, des données ou d'autres informations, comme illustré en Fig. 1 et Fig. 3.

Par ailleurs, la partie de boitier avant 31 comprend, en façade 3-1, c'est-à-dire du côté externe du boitier 3 comme illustré en Fig. 2, un moyens d'affichage 19, telle un écran ou afficheur 19-1, et, du côté interne 3-2, des moyens d'alarme 35 aptes à déclencher une alarme sonore, typiquement un dispositif à hautparleur ou analogue, tel un buzzer, porté ici par la carte électronique 34, et préférentiellement une alarme visuelle, telle un signal lumineux ou une information d'alerte affichée sur par les moyens d'affichage 19, tel l'écran 19-1.

Les moyens d'affichage 19 et les moyens d'alarme sonore 35 sont pilotés par les moyens de pilotage 4. De préférence, l'écran 19-1 est en couleurs et sert à visualiser différentes informations.

Le boitier 3 comprend par ailleurs plusieurs voies 16 ; 16-1...16-4, c'est-à-dire des liaisons électriques, destinées au raccordement de moyens de mesure 5, typiquement des capteurs de pression 5-1, 5-2, 5-3 servant à fournir des mesures de pression mesurées ou déterminée par ces moyens de mesure 5, aux moyens de pilotage 4 qui les traitent. Eventuellement, on peut aussi prévoir un dispositif de surveillance 15 de la production de vide par la pompe à vide 13 ou par l'une des autres sources de gaz 11, 12, i.e. PSA ou autre.

Plus précisément, les capteurs de pression 5-1, 5-2, 5-3 faisant office de moyens de mesure 5 sont agencés sur les différentes lignes d'acheminement de fluide 14-1, 14-2, 14-3 du réseau 14 de manière à pouvoir mesurer la pression des fluides circulant dans ces différentes lignes 14-1, 14-2, 14-3.

Ces capteurs de pression 5-1, 5-2, 5-3 sont reliés électriquement aux moyens de pilotage 4 par les liaisons 6 électriques, tels des câbles électriques ou analogue, et des moyens de raccordement électrique, tels des connecteurs électriques, par exemple un bornier ou analogue comprenant plusieurs emplacements de raccordement, et afin de leur fournir les mesures de pression opérées, i.e. valeurs ou signaux. A titre d'exemple, on peut utiliser un capteur de pression analogique 0-16 bars de marque Huba^{®}.

Autrement dit, les moyens de pilotage 4 sont configurés pour opérer une surveillance de la pression dans les lignes d'acheminement de fluide 14-1-14-3 en traitant les mesures de pression provenant des capteurs de pression 5 et ainsi pouvoir détecter toute pression inadéquate, i.e. excessive ou insuffisante.

De façon analogue, la pompe à vide 13 servant à produire le vide, c'est-à-dire la dépression servant à l'aspiration, peut être équipée d'un dispositif de surveillance 15, qui renvoie une synthèse des défauts de l'équipement (i.e. pannes ou anomalies), relié, lui aussi, aux moyens de pilotage 4, via une voie dédiée 6-4, pour leur fournir des informations de fonctionnement de la pompe 13 afin de pouvoir détecter tout dysfonctionnement de cette pompe à vide 13 et déclencher une (ou des) alarme en réponse à cette détection.

Les mesures de pression provenant des moyens de mesure 5 sont traitées informatiquement par les moyens de pilotage 4, en particulier par le (ou les) processeur(s) 4-1, pour détecter tout problème de pression ou dysfonctionnement éventuel du réseau 14, c'est-à-dire des lignes d'acheminement de fluide 14-1, 14-2, 14-3, ou de la pompe à vide 13, par exemple des pressions inadéquates, i.e. non-conformes, typiquement des pressions excessives ou insuffisantes, et déclencher ensuite une alarme sonore et/ou une alarme visuelle en réponse à la détection par exemple d'une chute de pression dans l'une des conduites de gaz du réseau 14 ou un dysfonctionnement de la pompe à vide 13, afin d'alerter le personnel hospitalier et lui permettre de prendre des mesures appropriées.

Des moyens d'alimentation électrique 20, tel que le secteur (110/220 V), alimentent électriquement les différents composants de l'installation 1 ayant besoin de courant électrique pour fonctionner, en particulier les moyens de pilotage 4, les différents capteurs 5, le dispositif de surveillance 15, la pompe à vide 13....

Afin que les moyens de pilotage 4 puissent remplir leur fonction, il est impératif que le boîtier de surveillance 3 soit programmé ou paramétré correctement, lors de sa mise en service notamment, afin d'assurer une conformité de l'installation, notamment en termes de sécurité. En particulier, les seuils de pression d'alarme, c'est-à-dire les seuils de pression haute et basse, doivent être définis avec précision, ainsi que d'autres paramètres de configuration, comme expliqué ci-après.

Pour ce faire, chaque voie 16 du boîtier 3 est paramétrée au sein du boîtier de surveillance 3, c'est-à-dire que des paramètres de configuration spécifiques associés à chacune des différentes voies 16 sont mémorisés au sein des moyens de mémorisation 4-2.

Le paramétrage, c'est-à-dire les paramètres de configuration, des différentes voies 16 du boîtier de surveillance 3 va varier selon le fluide surveillé par chacune de ces voies 16 et/ou, éventuellement selon le type d'utilisation, par exemple surveillance de la production ou surveillance de l'alimentation d'un service.

En général, un boîtier de surveillance 3 d'une installation 1 selon l'invention peut comprendre jusqu'à 8 à 10 voies différentes.

Pour ce faire, on définit et on mémorise au sein des moyens de mémorisation 4-2, un ensemble (Ei) de plusieurs (i≥2) paramètres de configuration spécifiques, à savoir typiquement :
- le type de fluide, typiquement oxygène, air, vide, voire du protoxyde d'azote (N₂O), du CO₂ ou autre,
- les seuils haut et/ou bas de pression d'alarme correspond au type de fluide considéré,
- le type de capteur utilisé, c'est-à-dire les caractéristiques du capteur relié à la voie 16 considérée, et/ou
- de préférence, le type de service hospitalier alimenté par le fluide considéré, par exemple cardiologie......

Chaque ensemble (Ei) de paramètres de configuration est alors attribué spécifiquement à l'une ou l'autre des voies 16 considérées. Il est donc spécifique, (i.e. dédié et/ou associé) d'une des (i=4) voies 16, par exemple l'une des 4 voies 16 schématisées en Fig. 1.

Ainsi, on a par exemple :
- un premier ensemble de paramètres de configuration (E₁) est spécifique de la première voie 16-1 qui est reliée (via une première liaison électrique 6-1) au premier capteur de pression 5-1 mesurant la pression d'oxygène dans la ligne d'acheminement d'oxygène 14-1 alimentée par la source d'oxygène 11 ;
- un deuxième ensemble de paramètres de configuration (E₂) est spécifique de la deuxième voie 16-2 qui est reliée (via une deuxième liaison électrique 6-2) au deuxième capteur de pression 5-2 mesurant la pression d'air médical dans la ligne d'acheminement d'air médical 14-2 alimentée par la source d'air médical 12 ; et
- un troisième ensemble de paramètres de configuration (E₃) est spécifique de la troisième voie 16-3 qui est reliée (via une troisième liaison électrique 6-3) au troisième capteur de pression 5-3 mesurant la dépression (i.e. niveau de vide) dans la ligne d'acheminement de vide 14-3 reliée à la pompe à vide 13.

Les liaisons électriques 6-1 à 6-3 sont typiquement des câbles électriques ou analogues permettant d'acheminer les signaux de mesure, qu'ils soient analogiques ou numériques.

De même, la quatrième voie 6-4 est définie par un ensemble de paramètres de configuration spécifiques permettant une surveillance de production via un capteur de fonctionnement de la pompe à vide.

Les ensembles de paramètres de configuration (Ei) sont mémorisés dans les moyens de mémorisation 4-2 faisant préférentiellement partie des moyens de pilotage 4 et coopérant avec ceux-ci, telle une mémoire flash ou analogue.

A titre d'exemple, le Tableau 1 ci-après donne des valeurs représentatives de plusieurs paramètres de tels ensembles de paramètres de configuration (E) mémorisés pour les différentes voies 16 (n°16-1 à 16-8) d'un boîtier de surveillance 3 à 8 voies.

**Tableau 1**

| Voie n° | Type de gaz | Type de capteur | Seuil de pression basse | Seuil de pression haute |
|---|---|---|---|---|
| 16-1 | O₂ | 0 à 16 bar | 3.8 bar | 5.8 bar |
| 16-2 | N₂O | 0 à 16 bar | 3.4 bar | 5 bar |
| 16-3 | Air médical | 0 à 16 bar | 3.6 bar | 5.4 bar |
| 16-4 | Air SEGA | 0 à 16 bar | 4 bar | 6 bar |
| 16-5 | Air 800 | 0 à 16 bar | 6.4 bar | 9.6 bar |
| 16-6 | CO₂ | 0 à 16 bar | 3.6 bar | 5.4 bar |
| 16-7 | Vide médical | 0 à -0.900 bar | -0.34 bar | 0 bar |
| 16-8 | Contacteur tout ou rien de la pompe à vide | Marche / Arrêt | - | - |

Comme on le voit, chaque voie 16-1 à 16-7 est associée à un ensemble de paramètres de configuration (Ei) comprenant un type de gaz, un type de capteur et des seuils de pression basse et haute définissant les niveaux ou pressions d'alerte ou d'alarme. Ainsi, en sélectionnant une voie donnée, les paramètres qui lui sont associés, sont immédiatement retrouvés dans les moyens de mémorisation 4-2, ce qui évite tout risque d'erreur qui peut se produire en cas de saisie manuelle.

La voie 16-8 est quant à elle associée au contacteur 15 de type tout ou rien de la pompe à vide 13 et sert à surveiller le bon fonctionnement de la production de vide, comme expliqué ci-avant.

De plus, les ensembles de paramètres Eᵢ peuvent être avantageusement regroupés en groupes Gₙ (n>1) comprenant chacun plusieurs ensembles de paramètres (Eᵢ). Les groupes Gₙ sont aussi mémorisés par les moyens de mémorisation 4-2.

Ainsi, lors de la mise en service du boîtier de surveillance 3 de l'installation 1 de Fig. 1, après avoir raccordé par exemple les voies 16 aux moyens de mesure 5, typiquement aux capteurs de pression 5-1 à 5-3, voire aussi au dispositif de surveillance 15 de la pompe à vide 13, comme expliqué ci-avant, l'opérateur n'a qu'à sélectionner au niveau du boitier de surveillance 3, via des moyens de sélection 18, tels un ou des boutons ou touches de sélection, le groupes Gₙ désiré parmi les groupes Gₙ.

Ceci permet à l'utilisateur de paramétrer rapidement, c'est-à-dire en une fois, toutes les voies 16 concernées auxquelles sont attribués les paramètres des ensembles de paramètres Eᵢ du groupe Gₙ sélectionné. Par exemple, en sélectionnant le groupe G1, l'utilisateur va attribuer les ensembles de paramètres E1, E2, E3 aux 3 voies concernées, par exemple les voies 16-1 à 16-3 de Fig. 1.

Un tel boitier permet d'éviter les erreurs de paramétrage lors de la mise en route du boîtier 3, en particulier lorsque l'opérateur sélectionne un groupe Gn formé de plusieurs ensembles de paramètres Eᵢ dédiés à des voies spécifiques 16 qui vont alors être paramétrées simultanément sur la base des paramètres mémorisés, ce qui évite les erreurs.

Fig. 2 représente un schéma d'un mode de réalisation de la face avant ou façade 3-1 d'un boîtier 3 selon l'invention.

On voit l'écran d'affichage 19-1, de préférence en couleurs qui affiche ici différentes informations, telles que le nom du fluide 190 de chaque voie, e.g. ici oxygène, air, vide... ; la valeur de (dé)pression 191 mesurée pour chaque fluide (en bar) ; un ou des pictogrammes 192 illustrant un état des différentes voies, en particulier pression « normale » ou « alarme » en cas de pression excessive ou insuffisante, c'est-à-dire un pictogramme d'alarme visuelle....

Selon l'invention, afin de permettre à un opérateur de réaliser tous les raccordements en évitant le déclenchement d'une alarme sonore, on prévoit des moyens de sélection de mode 18, agencés dans le compartiment interne 32 du boitier 3, de préférence dans la partie avant 31 du boitier 3, lesquels sont accessibles lorsque le boitier est ouvert, et sont actionnables par l'utilisateur pour activer, i.e. sélectionner, un mode de fonctionnement donné parmi plusieurs modes possibles, à savoir au moins un mode dit « de fonctionnement normal » et un mode dit « de maintenance ».

Les moyens de sélection de mode 18 comprennent un bouton, tel un bouton-poussoir, un contacteur basculant (switch) ou analogue. Avantageusement, les moyens de sélection de mode 18 sont agencés sur la carte électronique 34 faisant partie des moyens de pilotage 4 ou ailleurs.

Lorsque l'utilisateur actionne les moyens de sélection de mode 18, par exemple bascule un contacteur ou « switch », un signal électrique peut être émis ou à l'inverse interrompu (e.g. fermeture ou ouverture d'un circuit électrique) pour signaler cet actionnement aux moyens de moyens de pilotage 4, typiquement au processeur 4-1, lesquels prennent alors en compte le passage du mode « de fonctionnement normal » au mode « de maintenance », ou inversement.

Ainsi, lorsque les moyens de sélection de mode 18 sont actionnés pour activer ou passer en mode « de fonctionnement normal », les moyens de pilotage 4 peuvent activer les moyens d'alarme 35 de manière à pouvoir déclencher une alarme sonore et préférentiellement une alarme visuelle en cas de détection d'une pression inadéquate dans l'une ou plusieurs des lignes d'acheminement de fluide 14-1 à 14-3, alors qu'à l'inverse, en mode « de maintenance », les moyens de pilotage 4 agissent sur les moyens d'alarme 35 pour empêcher tout déclenchement d'alarme sonore tant que le mode dit « de maintenance » est activé.

Toutefois, il peut être souhaitable que les moyens de pilotage 35 puissent continuer à opérer une surveillance de la pression dans les lignes d'acheminement de fluide 14-1 à 14-3, lorsque le mode « maintenance » est activé de manière à pouvoir déclencher ou délivrer une alarme visuelle pour signaler à l'utilisateur que le boitier 3 est en mode « maintenance » ou qu'une pression inadéquate a été détectée par les moyens de pilotage 35.

Passer le boitier 3 en mode « de maintenance » permet d'éviter des déclenchements d'alarme sonore intempestifs, notamment lorsque l'opérateur opère un branchement des liaisons électriques, un paramétrage des voies ou autre.

Par ailleurs, la partie avant 31 du boitier 3 comprend, en façade avant 3-1, des touches de contrôle 21, 22 à actionnement par pression digitale de l'utilisateur, à savoir une première touche de contrôle 21 et une seconde touche de contrôle 22.

Ces touches de contrôle 21, 22 permettent à l'utilisateur, lorsque le mode « de fonctionnement normal » a été activé, de stopper une alarme sonore ayant été déclenchée par les moyens d'alarme sonore 35, c'est-à-dire d'acquitter une alarme, ou de tester un bon fonctionnement des moyens d'alarme sonore 35, tel un buzzer, ou, à l'inverse, lorsque le mode « de maintenance » a été activé, permettre à l'utilisateur de sélectionner ou fixer au moins un paramètre de configuration ou encore de choisir une langue d'affichage.

Préférentiellement, les touches de contrôle 21, 22 assurent chacune une double fonction.

Par exemple, la première touche de contrôle 21 permet à l'utilisateur de stopper une alarme sonore ayant été déclenchée par les moyens d'alarme sonore 35 lorsque le mode « de fonctionnement normal » est activé et par ailleurs de sélectionner ou fixer au moins un paramètre de configuration lorsque le mode « maintenance » est activé, alors que la seconde touche de contrôle 22 permet, quant à elle, à l'utilisateur de tester un bon fonctionnement des moyens d'alarme sonore 35 lorsque le mode « de fonctionnement normal » est activé et, par ailleurs, de choisir une langue d'affichage lorsque le mode « maintenance » est activé, par exemple le français, l'anglais, l'espagnol, l'italien, l'allemand, le portugais, l'arabe ou une autre langue.

Avantageusement, lorsque le mode « maintenance » est activé, les moyens de pilotage 4 commandent les moyens d'alarme 35 pour délivrer un signal sonore, i.e. un signal de rappel, tel un « bip sonore » ou analogue, à une fréquence donnée, par exemple toutes les 1 à 10 minutes, typiquement toutes les 3 à 7 minutes, de manière à rappeler à l'opérateur que le boitier 3 est en mode « de maintenance ».

Préférentiellement, le boitier 3 comprend aussi une (des) alarme visuelle qui est préférentiellement activée en même temps que l'alarme sonore, par exemple en cas de détection d'une pression inadéquate ou non-conforme, typiquement un signal lumineux et/ou un message d'alarme affichés sur l'écran 19-1.

Avantageusement, le boitier de surveillance 3 comprend aussi des moyens de verrouillage (non montrés) permettant de verrouiller la partie de boitier avant 30 sur la partie de boitier arrière 31 pour empêcher une personne non-habilitée d'ouvrir le boitier 3 en passant la partie de boitier avant 30 en position d'ouverture, donc d'empêcher tout accès non autorisé au compartiment interne 32 définit par les deux demi-boitiers 30, 31. Lorsqu'ils sont déverrouillés, les moyens de verrouillage libèrent la partie avant de boitier 30 et autoriser ainsi son passage en position d'ouverture.

Les moyens de verrouillage comprennent typiquement un mécanisme d'ouverture et/ou de fermeture configuré pour être actionnable au moyen d'un moyen de déverrouillage dédié permettant d'opérer un verrouillage ou un déverrouillage. Le moyen de déverrouillage peut comprendre un outil, une clé ou analogue, ou alternativement, un système de verrouillage/déverrouillage par code confidentiel ou biométrique. Ceci permet de renforcer la sécurité du boitier 3.

Un tel boitier 3 est particulièrement bien adapté à une utilisation au sein d'un établissement hospitalier afin d'y opérer une surveillance du réseau de fluides médicaux, c'est-à-dire le réseau de gaz ou de vide médical (i.e. dépression) et de pouvoir avertir en cas de détection d'un dysfonctionnement engendrant une pression inadéquate au sein du réseau, c'est-à-dire trop faible ou trop élevée.

## Revendications

1. **Installation** (1) d'acheminement de fluide dans un établissement hospitalier, comprenant :
- des lignes d'acheminement de fluide (14-1, 14-2, 14-3) comprenant chacune un capteur de pression (5), et
- un boitier de surveillance de fluide (3) comprenant une partie de boitier arrière (30) et une partie de boitier avant (31) définissant entre elles un compartiment interne (32) du boitier (3), et des moyens d'alarme (35), dans lequel :
∘ ladite partie de boitier avant (31) est montée pivotante (XX) sur la partie de boitier arrière (30) entre au moins : une position d'ouverture dans laquelle un utilisateur a accès au compartiment interne (32) et une position de fermeture dans laquelle l'utilisateur n'a pas accès au compartiment interne (32),
∘ et la partie de boitier avant (31) comprend :
▪ du côté interne (3-2), des moyens de pilotage (4) à processeur (4-1) configurés pour opérer une surveillance de la pression dans les lignes d'acheminement de fluide (14-1, 14-2, 14-3) en traitant les mesures de pression provenant des capteurs de pression (5) et
▪ en façade (3-1), au moins une touche de contrôle (21, 22) à actionnement par pression digitale de l'utilisateur,
**caractérisé en ce que** :
- la partie de boitier avant (11) comprend en outre des moyens de sélection de mode (18) agencés dans le compartiment interne (32) du boitier (3) et actionnables par l'utilisateur pour activer :
▪ un mode dit « de fonctionnement normal », dans lequel les moyens de pilotage (4) sont configurés pour commander les moyens d'alarme et déclencher au moins une alarme sonore en cas de détection d'une pression inadéquate dans l'une ou plusieurs des lignes d'acheminement de fluide (14-1, 14-2, 14-3), ou
▪ un mode dit « de maintenance », dans lequel les moyens de pilotage (4) sont configurés pour contrôler les moyens d'alarme (35) pour empêcher tout déclenchement d'alarme sonore tant que le mode dit « de maintenance » est activé, et
- ladite au moins une touche de contrôle (21, 22) est configurée pour :
▪ lorsque le mode « de fonctionnement normal » a été activé, permettre à l'utilisateur de stopper une alarme sonore ayant été déclenchée par les moyens d'alarme (35) ou de tester un bon fonctionnement des moyens d'alarme (35), et
▪ lorsque le mode « de maintenance » a été activé, permettre à l'utilisateur de sélectionner ou fixer au moins un paramètre de configuration.

2. Installation selon la revendication 1, **caractérisée en ce que** les moyens de sélection de mode (18) comprennent un bouton, un contacteur basculant ou analogue.

3. Installation selon l'une des revendications précédentes, **caractérisée en ce que** les moyens de sélection de mode (18) sont agencés sur une carte électronique (34) faisant partie des moyens de pilotage (4).

4. Installation selon l'une des revendications précédentes, **caractérisée en ce qu'**au moins deux touches de contrôle (21, 22) à actionnement par pression digitale de l'utilisateur sont agencées sur la façade (3-1) du boitier (3), comprenant une première touche de contrôle (21) et une seconde touche de contrôle (22).

5. Installation selon les revendications 1 et 4, **caractérisée en ce que** la première touche de contrôle (21) est configurée pour permettre à l'utilisateur de stopper une alarme sonore ayant été déclenchée par les moyens d'alarme (35) lorsque le mode « de fonctionnement normal » est activé et/ou sélectionner ou fixer au moins un paramètre de configuration lorsque le mode « maintenance » est activé.

6. Installation selon les revendications 1 et 4, **caractérisée en ce que** la seconde touche de contrôle (22) est configurée pour permettre à l'utilisateur de tester un bon fonctionnement des moyens d'alarme (35) lorsque le mode « de fonctionnement normal » est activé et/ou de choisir une langue d'affichage lorsque le mode « maintenance » est activé.

7. Installation selon les revendications 1 et 4, **caractérisée en ce que** lorsque le mode « maintenance » est activé, les moyens de pilotage (4) sont configurés pour commander les moyens d'alarme (35) pour délivrer un signal sonore à une fréquence donnée, typiquement toutes les 3 à 7 minutes, de manière à indiquer à l'utilisateur que le boitier (3) est en mode « maintenance ».

8. Installation selon l'une des revendications 1, 5 ou 7, **caractérisée en ce que** les moyens d'alarme (35) comprennent un buzzer.

9. Installation selon la revendication 1, **caractérisée en ce que** la partie de boitier avant (31) comprend des moyens d'affichage (19) agencées en façade (3-1) et les moyens d'alarme (35) coopèrent avec les moyens d'affichage (19) pour afficher ou déclencher une alarme visuelle (192), simultanément au déclenchement d'une alarme sonore.

10. Installation selon la revendication 1, **caractérisée en ce que** les moyens de sélection de mode (18) sont agencés dans la partie de boitier avant (31) du côté du compartiment interne (32) du boitier (3).

11. Installation selon l'une des revendications 1 ou 7, **caractérisée en ce que** les moyens de pilotage (35) sont configurés pour continuer à opérer une surveillance de la pression dans les lignes d'acheminement de fluide (14-1 à 14-3), lorsque le mode « maintenance » est activé de manière à pouvoir déclencher ou délivrer une alarme visuelle pour signaler à l'utilisateur qu'une pression inadéquate a été détectée par les moyens de pilotage (35).

12. Installation selon la revendication 1, **caractérisée en ce que** ledit au moins un paramètre de configuration est choisi parmi un type de fluide, un type de capteur de pression, un seuil de pression basse et un seuil de pression haute.

13. Installation selon la revendication 1, **caractérisée en ce que** ladite au moins une touche de contrôle (21, 22) est configurée pour permettre en outre à l'utilisateur de choisir une langue d'affichage lorsque le mode « maintenance » est activé.

14. Installation selon la revendication 9, **caractérisée en ce que** les moyens d'affichage (19) agencées en façade (3-1) comprennent un écran à affichage en couleurs.

15. Installation selon la revendication 1, **caractérisée en ce que** les lignes d'acheminement de fluide (14-1, 14-2, 14-3) sont choisies parmi au moins une ligne d'oxygène, une ligne d'air et une ligne de vide, de préférence le boitier de surveillance de fluide (3) et les lignes d'acheminement de fluide (14-1, 14-2, 14-3) sont agencés sur une ou des parois de l'établissement hospitalier.
